# EUROPEAN PATENT APPLICATION

(11) **EP 4 792 735 A2**
(43) Date of publication of application: **19.08.2026**
(21) Application number: 26189775.5
(22) Date of filing: 28.07.2023
(51) Int. Cl.: A61B 5/00

(54) **METHOD AND SYSTEM FOR SSEP (SOMATOSENSORY EVOKED POTENTIALS) WITH MONITORABLE BASELINE WAVEFORM DETERMINATION**

(30) Priority: 28.07.2022 US 202263393230 P
(62) Document number(s) of the earlier application(s) in accordance with Art. 76 EPC: 23761272.6 / 4 561 430
(71) Applicant: Alphatec Spine, Inc., Carlsbad, CA 92008 (US)
(72) Inventor: VINCENT, Kevin, Carlsbad, 92008 (US)
(74) Representative: ip21 Ltd

(57) **Abstract**

Disclosed is a method for execution by an SSEP (Somatosensory Evoked Potentials) system. The method involves acquiring at least one SSEP recording from a subject, and determining if the baseline potential is monitorable based on the at least one SSEP recording. The method also involves acquiring ongoing SSEP recordings from the subject, comparing the ongoing SSEP potentials to the monitorable baseline potential, and upon the ongoing SSEP potentials deviating from the monitorable baseline potential according to a defined criteria, executing an alert. This can allow a medical worker to decide whether to take any corrective action, such as repositioning the subject, with a goal of preventing or mitigating iatrogenic injury to a nervous system of the subject. The SSEP system can be substantially automated, such that there is little reliance on discretion by the medical worker. Also disclosed is a SSEP system configured to implement the method summarised above.

## Description

### CROSS REFERENCE TO RELATED APPLICATION

A claim for priority to the July 28, 2022 filing date of U.S. provisional patent application no. 63/393,230, titled METHOD AND SYSTEM FOR SSEP (SOMATOSENSORY EVOKED POTENTIALS) WITH MONITORABLE BASELINE WAVEFORM DETERMINATION ("the '230 Provisional Application") is hereby made pursuant to 35 U.S.C. § 119. The entire disclosure of the '230 Provisional Application is incorporated herein by reference.

### FIELD OF THE DISCLOSURE

This disclosure relates to evoked potential (EP) monitoring systems for avoiding or mitigating iatrogenic nerve injuries during surgery.

### BACKGROUND

Short Latency Somatosensory Evoked Potentials (SSEP) are small sensory potentials (also referred to as responses or waveforms) that are time locked to a stimulus (usually electrical) of a peripheral or cranial nerve. Due to their small size they are typically identified by averaging together multiple fixed duration recordings that are time locked to multiple stimuli to improve signal to noise ratio. They are often recorded from a head or neck area. Monitoring subjects using SSEP during several types of surgery has been shown to provide early identification of iatrogenic or other injury to nervous system structures. Early identification of these physiological changes in nervous system function allows intervention to avoid or mitigate potential injury.

Such monitoring generally involves highly trained technologists under physician supervision using complicated, multichannel amplifier and display equipment. Unfortunately, such personnel and equipment are limited in their availability, involve pre-booking, may vary in quality, and are costly.

Collection of baseline recordings and identifying a baseline potential is fundamental to SSEP interoperative monitoring. The baseline potential is a reference that subsequently collected SSEP potentials can be compared to throughout the duration of the monitoring. The baseline recording is typically collected following anesthesia induction and prior to when surgical intervention has begun.

Most existing SSEP systems primarily rely on a medical worker (e.g. technician/neurologist) to interpret the electrical potentials to ascertain whether a baseline potential is present and monitorable within the baseline recordings and, by comparison, when a significant change in the ongoing SSEP potential (those recorded after the baseline is established) occurs. Also, while some potentials may be initially identifiable at baseline recording evaluation, they may be too small or inadequate to be reliably indicate change due to impending injury. This process is complicated, delayed, and is subject to the bias, variable expertise, and focus of the medical worker. Improper classification of a baseline potential as present, absent, or present but inadequate for monitoring can result in subsequent over or under recognition of nerve function change and fail to inform the user of potential nerve injury or suggest possible injury when none is present.

It is an object of the disclosure to improve upon the conventional approaches to address or mitigate some or all of the shortcomings noted above.

### SUMMARY OF THE DISCLOSURE

Disclosed is a method for execution by an SSEP (Somatosensory Evoked Potentials) system. The method involves acquiring at least one SSEP recording from a subject, and determining a monitorable baseline potential based on the at least one SSEP recording. The method also involves acquiring ongoing SSEP recordings from the subject, comparing the ongoing SSEP potentials to the baseline potential, and determining deviation of the ongoing SSEP potential from the baseline potential according to a defined criteria, and executing an alert if a significant change is identified.

In this way, a medical worker can be prompted to possibly take corrective action(s), such as, but not limited to, repositioning the subject or reducing a surgical retractor, with a goal of preventing or mitigating injury to the nervous system of the subject. Although the medical worker can make a final decision as to whether to take any corrective action, the SSEP system can substantially and more accurately inform or prompt such a decision or action. This improvement is made possible in part by innovations in how the SSEP system identifies whether there is a monitorable baseline potential.

In some implementations, acquiring at least one SSEP baseline recording involves acquiring two component SSEP recordings based on two corresponding independent sets of SSEP data, and the baseline potential is identified within both recordings and the grand ensemble average of the independent sets of SSEP data. The baseline potential is deemed monitorable or non-monitorable based on features of the two recordings as well as the recording created from the grand ensemble average. This can enable the SSEP system to reliably identify whether there is a monitorable baseline potential.

In some implementations, for the baseline SSEP recording, calculating or identifying the primary peak of the potential response involves identifying candidate peaks within the recording in an upright representation and an inverted representation of the baseline SSEP recording, and identifying which candidate peak has greatest prominence based on how much the candidate peak stands out due to its intrinsic height and its location relative to other candidate peaks, wherein the candidate peak with greatest prominence is used in the comparing of the baseline SSEP potential to the ongoing SSEP potentials.

In some implementations, for each ongoing SSEP recording, identifying the primary peak of the potential involves identifying candidate peaks with the same polarity as the primary peak in the baseline SSEP recordings. In some implementations, this may mean selecting the primary peak from the candidate peaks based on features of the peak and information from the baseline SSEP waveform and previous ongoing SSEP waveforms. In some implementations, this may mean selecting the candidate peak based on either the proximity of the candidate peak relative to the primary peak of the identified potential in the previous ongoing recording or based on the prominence of the candidate peak relative to other peaks. This can enable the SSEP system to reliably compare and identify the ongoing SSEP potential to the baseline SSEP potential.

Also disclosed is a non-transitory computer readable medium having recorded thereon statements and instructions that, when executed by a processor of an SSEP system, configure the processor to implement a method as summarized above.

Also disclosed is a baseline optimization state where setting the baseline is adaptive.

Also disclosed is an SSEP system. The SSEP system has stimulating electrodes configured to elicit responses from peripheral or cranial nerve that traverse a subject through their nervous system, and recording electrodes configured to sense the electric potentials upon traversing the nervous system. The SSEP system also has a nerve injury detection device coupled to the stimulating electrodes and the recording electrodes and configured to implement a method as summarized above.

Other aspects and features of the present disclosure will become apparent, to those ordinarily skilled in the art, upon review of the following description of the various embodiments of the disclosure.

### BRIEF DESCRIPTION OF THE DRAWINGS

Embodiments will now be described with reference to the attached drawings in which:
Figure 1 is a block diagram of an SSEP (Somatosensory Evoked Potentials) system for monitoring a subject;
Figure 2 is a flowchart of a method of monitoring the subject for risk of nerve injury;
Figure 3 is a flowchart of a method of identifying the primary and reference peaks of a potential in a baseline SSEP recording;
Figures 4 and 5 are graphs of a baseline SSEP recording in an upright representation and an inverted representation;
Figure 6 is a flowchart of a method of determining whether a baseline recording contains a monitorable baseline potential;
Figure 7 is a graph of baseline SSEP recordings;
Figure 8 is a collection of charts relating to baseline SSEP recordings with a monitorable potential;
Figure 9 is a collection of charts relating to baseline SSEP recordings with a non-monitorable potential;
Figure 10 is a collection of charts relating to baseline SSEP recordings with a monitorable potential and improving response quality as more single trials are collected;
Figure 11 is a flowchart of a method of identifying the primary and reference peaks of a potential in an ongoing SSEP recording;
Figure 12 is a graph of baseline and ongoing SSEP recordings from a case example with a monitorable baseline potential displayed in a 'waterfall' view;
Figure 13 is a graph of baseline and ongoing SSEP recordings from a case example with a non-monitorable baseline potential and a user initiated override of the baseline classification displayed in a 'waterfall' view;
Figure 14 is a graph of SSEP recordings relating to a case example with a monitorable baseline potential;
Figure 15 is a graph of SSEP recordings relating to a case example with a non-monitorable baseline potential; and
Figure 16 is a graph of SSEP recordings relating to a case example with a non-monitorable baseline potential.

### DETAILED DESCRIPTION OF EMBODIMENTS

It should be understood at the outset that although illustrative implementations of one or more embodiments of the present disclosure are provided below, the disclosed systems and/or methods may be implemented using any number of techniques. The disclosure should in no way be limited to the illustrative implementations, drawings, and techniques illustrated below, including the exemplary designs and implementations illustrated and described herein, but may be modified within the scope of the appended claims along with their full scope of equivalents.

Evoked potentials are the electrical signals generated by the nervous system in response to sensory stimuli. In other words, an evoked potential is the neurophysiological response to an electronic stimuli. Auditory, visual, and somatosensory stimuli are used commonly for clinical evoked potential studies. SSEPs consist of a series of waves that reflect sequential activation of neural structures along the somatosensory pathways. While SSEPs can be elicited by mechanical stimulation, clinical studies use electrical stimulation of peripheral nerves, which gives larger and more robust responses. Selected nerves are typically stimulated with monophasic square pulses, 100-300 microseconds in duration. Stimuli are delivered by using either a constant voltage or a constant current stimulator.

SSEPs are typically not visible in the raw data recorded from surface electrodes. Signal averaging is used to extract the SSEPs from the other electrical signals picked up by the recording electrodes. The waveform resulting from the stimulation of a nerve is displayed as a plot of voltage against time and is characterised by measurements of post-stimulus latencies (in milliseconds) and amplitudes (in microvolts) of particular peaks. Recordings are a time series that show the measured electrical signal over time. Several characteristics of SSEPs can be measured, including peak latencies, component amplitudes, and waveform morphology. According to typical convention, deflections below the baseline are labelled positive and those above the baseline are negative. Standard identification of waveforms is by a letter designating the direction of the deflection followed by a number representing the latency of the waveform.

Peak latencies are typically consistent across subjects, whereas amplitudes show large intersubject variability. Interpretation of extra-operative diagnostic SSEP studies is predominantly based on peak latencies and measures derived from them, such as interpeak intervals and right-left differences. Component amplitudes are more consistent during repeated SSEP recordings in the same subject and may change sooner than latencies change, or in the absence of any latency changes, if the somatosensory pathways are damaged during surgery. Therefore, both peak latencies and component amplitudes should be measured and followed during intraoperative monitoring.

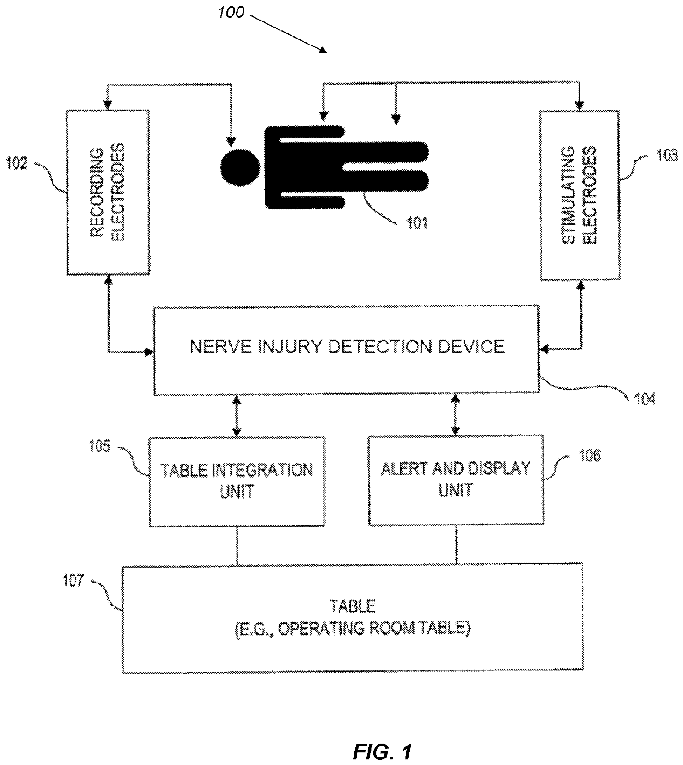

Figure 1, shown is a block diagram of an SSEP (Somatosensory Evoked Potentials) system 100 for monitoring a subject 101. The SSEP system 100 has one or more recording electrodes 102, one or more stimulating electrodes 103, and a NIDD (Nerve Injury Detection Device) 104. In some implementations, the SSEP system 100 also has a table integration unit 105, an alert and display unit 106, and a table 107. The SSEP system 100 may also have other components that are not shown. Note that the subject 101 is not part of the SSEP system 100.

In some implementations, the subject 101 is positioned on the table 107. The recording electrodes 102 are coupled to the subject 101, for example their head, neck, arms, legs, trunk, Erb's point and/or torso. The stimulating electrodes 103 are also coupled to the subject 101, for example their arms and/or legs. The stimulating electrodes 103 are configured to generate SSEP responses, which traverse the subject 101 through their nervous system, and are sensed by the recording electrodes 102. It is possible to detect iatrogenic injuries in the nervous system of the subject 101 based on the SSEP responses which are sensed by the recording electrodes 102. The SSEP baseline recording, ongoing SSEP recordings, and any derived features or information may be displayed on the alert and display unit 106 of the SSEP system.

Determining whether there is risk to a structure in the nervous system of the subject 101 involves technical difficulties in part because the SSEP potentials exhibit considerable variability between subjects and the SSEP recordings can include electrical noise and/or artifacts. To assess whether the SSEP potentials indicate risk to a structure in the nervous system of the subject 101, the ongoing SSEP potentials are compared to a baseline potential, which can be measured, identified, and classified as monitorable or non-monitorable as an initial step, for example prior to commencing surgery or other procedure on the subject 101. Depending on whether the ongoing SSEP potentials match or deviate from the monitorable baseline potential, an alert can be executed. The comparison of the ongoing SSEP potential to the baseline SSEP potential may be based on salient characteristics such as the evoked potential amplitude and/or latency. Further example details are provided below.

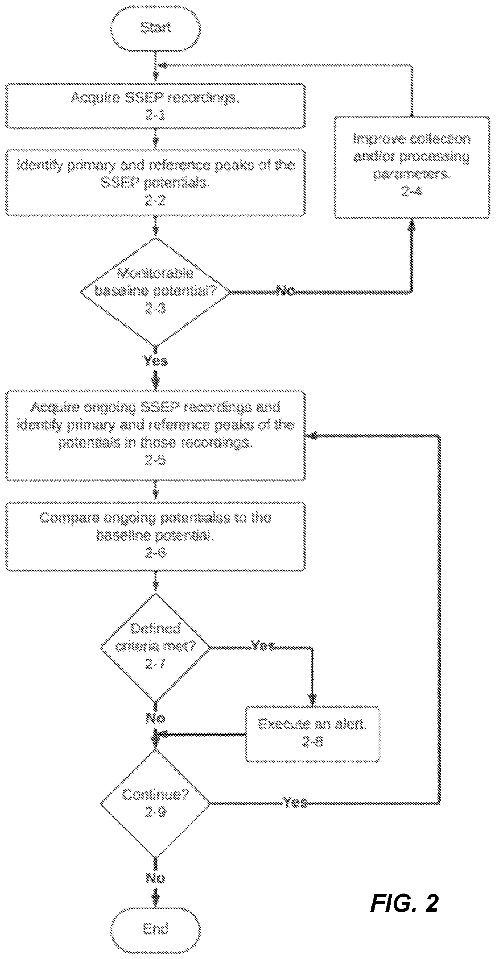

Referring now to Figure 2, shown is a flowchart of a method of monitoring the subject 101 for risk of nerve injury. This method can be executed by an SSEP system, for example the SSEP system 100 depicted in Figure 1. In particular implementations, the method is executed by the NIDD 104 of the SSEP system 100. More generally, the method of Figure 2 can be executed by any suitable SSEP system.

At step 2-1, the SSEP system 100 acquires SSEP recordings. For example, as noted above, the stimulating electrodes 103 can generate electric stimuli, which activate peripheral nerves in the subject 101 and send action potentials that traverse the subject 101 through their nervous system, and are sensed by the recording electrodes 102. The responses recorded after individual stimuli, or single trial responses, can be ensemble averaged to improve the signal-to-noise ratio of the evoked potential response. In this way, the SSEP system 100 acquires one or more SSEP recordings. Independent sets of SSEP data are taken in distinct trials and do not have overlapping single trials between two independent sets of SSEP data.

At steps 2-2 and 2-3, the SSEP system 100 determines, based on the SSEP recordings acquired at step 2-1, whether there is a monitorable baseline potential. Note that steps 2-1 to 2-3 can be repeated until a monitorable baseline potential is found. There are many ways that the SSEP system 100 can determine whether there is a monitorable baseline potential. In some implementations, the SSEP system 100 uses a machine learning algorithm to classify each baseline SSEP recording as having a monitorable or non-monitorable potential. In some implementations, when classifying the baseline potentials, the machine learning algorithm is a supervised machine learning algorithm where the features used by the algorithm are based on morphology and repeatability.

The morphology of evoked potentials can be variable across subjects, but there are some common/typical morphologies. There is also a range for expected latencies between stimulus and when the evoked potential appears in a recording given where on the subject 101 the stimulus is located (e.g. longer latency for stimulating at the ankle than the wrist due to a longer distance traveled). If deflections in a recording repeat at the same time relative to the electrical stimulus in subsequent, independent data sets, it is more likely that the response contains a real SSEP potential rather than artifact, noise or some other physiological or non-physiological source. A real SSEP potential with identifiable primary and reference peaks and sufficient amplitude above the noise floor should be monitorable. By comparing morphology features in isolation and over time it is possible to make an accurate classification. Further example details of how the SSEP system 100 can determine whether there is a monitorable baseline potential are provided later.

In some implementations, as shown at step 2-2, the SSEP system 100 identifies peaks of the SSEP potential. The SSEP system 100 can then use such peak marking to assess morphology and repeatability, with a goal of determining whether there is a monitorable baseline potential. There are many ways that the SSEP system 100 can identify the main peak and reference peak(s) of the SSEP potentials. In some implementations, the SSEP system 100 uses an algorithm to identity peaks based on the maximum value, minimum value, derivative or second derivative. In an exemplary implementation the SSEP system 100 uses the prominence of candidate peaks within a stimulation/recording site-specific analysis window to identify the primary peak. The prominence of a candidate peak measures how much the candidate peak/trough stands out due to its intrinsic height and its location relative to other candidate peaks/troughs. A low isolated peak in the signal can be more prominent than one that is higher but is an otherwise unremarkable member of a grouping of signal peaks. In some implementations, the SSEP system 100 may find the reference peak by finding the greatest maxima or minima with the opposite polarity of the primary peak in the region around the primary peak. Further example details of how the SSEP system 100 can identify primary and reference peaks of the baseline SSEP potential are provided later.

If the baseline classification algorithm determines there is not a monitorable baseline potential at step 2-3, the SSEP system 100 may, at step 2-4, alter the collection or processing parameters to improve the quality or monitorability of the SSEP potentials. The goal of this improvement is to enable the system to establish a monitorable baseline. For example the SSEP system 100 may increase the stimulus current administered by the stimulating electrodes 103, decrease the rate of stimulation by the stimulating electrodes 103, increase the number of single trials include in the ensemble average or reduce the amount of digital filtering applied to the SSEP recording. Improvement or optimization of these parameters may be driven by an output from the baseline classification algorithm or other features calculated from the SSEP recordings such as signal-to-noise ratio. The improvements to the collection and processing parameters may be automated so as to not require intervention by the user. Further example details of how the SSEP system 100 may improve or optimize collection and/or signal processing parameters in response to a non-monitorable classification are provided later.

Upon determining that there is a monitorable baseline potential, the SSEP system 100 can be used to monitor the subject 101 on an ongoing basis, for example during surgery or other medical procedure. Thus, at step 2-5 the SSEP system 100 acquires ongoing recordings and identifies peaks of potential in those recordings. The manner in which the primary and reference peaks are identified can be similar or identical as in step 2-2. In an exemplary embodiment, the SSEP system 100 uses parameters set when identifying the primary and reference peaks in the baseline potential and information from previous ongoing SSEP recordings to identify the peaks of interest in the ongoing SSEP potential. Then, the SSEP system 100 compares the ongoing potentials to the baseline potential at step 2-6. For example, the SSEP system 100 can compare amplitude and/or latency based on the primary and reference peaks that have been identified. In an exemplary implementation, the amplitude comparison between the baseline and ongoing potentials is the amplitude defined by the vertical distance between the primary and reference peaks, and the latency comparison is defined by the latency of the primary peak. Such comparison is performed in accordance with some defined criteria to assess whether the ongoing potentials are consistent with the baseline potential which may indicate a normal condition, or inconsistent with the baseline waveform which may indicate potential threat of nerve injury.

If at step 2-7 the defined criteria is met, then at step 2-8 the SSEP system 100 executes an alert, because there may be risk of iatrogenic nerve injury. In some implementations, the alert and display unit 106 of the SSEP system 100 generates the alert. There are many possibilities for the alert. The alert can for example include an auditory alert, a visual alert, and/or a haptic alert. The purpose of the alert is to inform a physician and/or other person that there may be risk of iatrogenic nerve injury to the subject 101. Such physician and/or other person could then decide whether to take any corrective action, such as repositioning the subject to prevent excessive pressure/tension or reduced blood supply to a nerve, with a goal of eliminating or mitigating the risk of nerve injury to the subject 101.

Note that steps 2-5 to 2-8 can repeat on an ongoing basis throughout the surgery or other medical procedure. However, if at step 2-9 the SSEP system 100 is to conclude the method, then the method ends. The method can conclude for example if input is received via the alert and display unit 106 of the SSEP system 100 to end the monitoring of the subject 101. Such input could for example be provided by the physician and/or other person after the surgery or other medical procedure has been completed, or for other reasons.

The defined criteria mentioned above for step 2-7 can for example include a defined decrease in amplitude (i.e. the vertical distance from the peak marker to the onset or offset marker) and/or a defined increase in latency (i.e. time from stimulus to the peak). During surgery or other procedure, the ongoing recordings can be updated on the alert and display unit 106 in a sliding window manner. For each updated ongoing recording, the SSEP system 100 can calculate the amplitude and the latency of the evoked potential (latency is the timing of the peak). The amplitude and latency of the ongoing potential are compared to the amplitude and latency of the baseline potential. Standard thresholds used in interoperative neuromonitoring to determine a significant change from baseline are a 50% reduction in amplitude of the ongoing potential relative to the baseline potential or a 10% longer latency (the peak occurs 10% later in time) in the ongoing potential relative to baseline. Other thresholds are possible. In some implementations, the SSEP system 100 applies an additional level of logic on top of those thresholds that is a 'voting' scheme used to reduce false positive alerts such as that disclosed in U.S. Patent No. 11,197,640. If a single ongoing potential exceeds the defined thresholds, that is considered a vote for an alert. The number of alert votes in a sliding window is to exceed the defined alert vote threshold before an alert is raised by the SSEP system 100 via the alert and display unit 106. For example, 80% of the previous 30 active traces (24/30) is to exceed threshold ('Y' votes) for the SSEP system 100 to raise an alert.

In some implementations, the voting process is asymmetrical, and different ratios of voting may be used for triggering (onset) and releasing (offset) a final alert to the user that nerve injury to the subject is possible or imminent. The alerting process examines the meta-data (the alerting vote) for the individual overlapping epochs. Since any real change in data will be sustained and always eventually deliver 100% (or close) Y/N votes for alert, the specificity of the alerting process can now be manipulated independent of the sensitivity of the system on the basis of the voting. For example, the user may alter the ratio to be less specific, involving only 30% or 50% of votes vs 80% of votes for more specificity. In this way, there can be a reduction in the number of false alerts without really altering the sensitivity of the process to detecting real sustained change. The voting ratio can lessen the impact of the noisy signals. In some implementations, SSEP recordings with wildly variant values can be discarded from the voting process, eliminating intermittent noise that escapes the frequency filters. Thus, some implementations have an improved way of handling the effects of noise in SSEP recordings. The effect of this noise is further nullified by the voting process itself.

There are many possibilities for the components of the SSEP system 100. In some implementations, the NIDD 104 includes a computer. In some implementations, the NIDD 104 is electrically, electronically, and/or mechanically coupled to the table integration unit 105 and/or the alert and display unit 106. In some implementations, the table integration unit 105 is mechanically and/or electronically coupled to the table 107 and/or NIDD 104. In some implementations, the table integration unit 105 is incorporated in the table 107 and/or NIDD 104. In some implementations, the alert and display unit 106 is mechanically and/or electrically coupled to the NIDD 104 and/or table 107. In some implementations, the alert and display unit 106 is incorporated in the table 107 and/or NIDD 104 or displayed on other screens available to the user.

As noted above, the NIDD 104 of the SSEP system 100 can include a computer. In some implementations, the NIDD 104 implements the methods described herein using software that is executed by a processor of such computer. According to an embodiment of the disclosure, there is provided a non-transitory computer readable medium having recorded thereon statements and instructions that, when executed by the processor, implement a method as described herein. The non-transitory computer readable medium can for example include an SSD (Solid State Drive), a hard disk drive, a CD (Compact Disc), a DVD (Digital Video Disc), a BD (Blu-ray Disc), a memory stick, or any appropriate combination thereof.

It is noted that non-software implementations are also possible and are within the scope of this disclosure. Other implementations can include additional or alternative hardware components, such as any appropriately configured FPGA (Field-Programmable Gate Array) and/or ASIC (Application-Specific Integrated Circuit), for example. More generally, the NIDD 104 of the SSEP system 100 can be implemented with any suitable combination of hardware, software and/or firmware.

Further example details are provided below regarding how the SSEP system 100 can identify the primary and reference peaks of the baseline SSEP potential, how the SSEP system 100 can determine whether there is a monitorable baseline potential, how the SSEP system 100 can improve the collection and/or processing of SSEP recordings, and how the SSEP system 100 can identify the primary and reference peaks of the ongoing SSEP potential so as to enable monitoring for nerve injury. It is to be understood that these example details are very specific, such that other implementations are possible and are within the scope of this disclosure.

### Identifying Peaks in the Baseline SSEP Recording

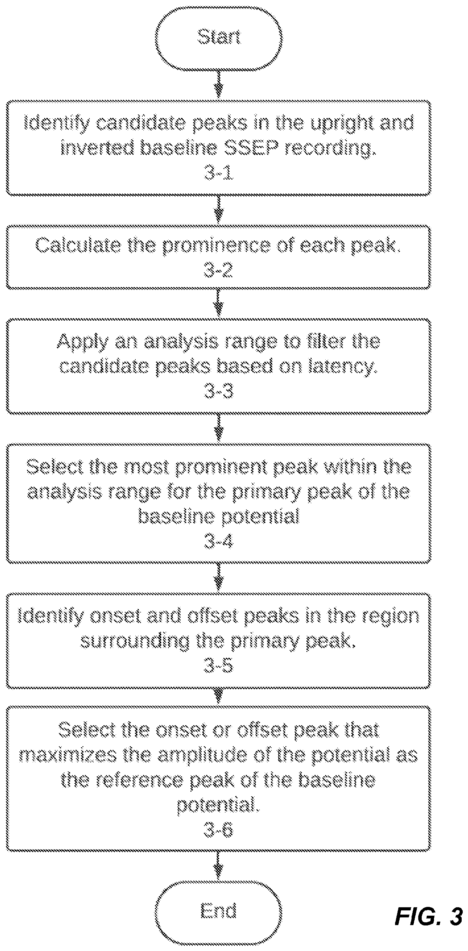

Referring now to Figure 3, shown is a flowchart of a method of identifying the relevant peaks of an evoked potential in a baseline SSEP recording. This method is an example implementation for step 2-2 of the method described above in relation to Figure 2. Thus, much like the method of Figure 2, the method of Figure 3 can be executed by an SSEP system, for example the SSEP system 100 depicted in Figure 1. In particular implementations, the method is executed by the NIDD 104 of the SSEP system 100. More generally, the method of Figure 3 can be executed by any suitable SSEP system.

Identifying the peaks of interest within the baseline SSEP recording is an important step in a NIDD 104. In the preferred embodiment, the prominence of a peak is used to identify the primary peak of the evoked potential. However, there are other possible embodiments. The selection of the primary peak could be based on the maximum value, maximum absolute value, slope or second derivative near the peak.

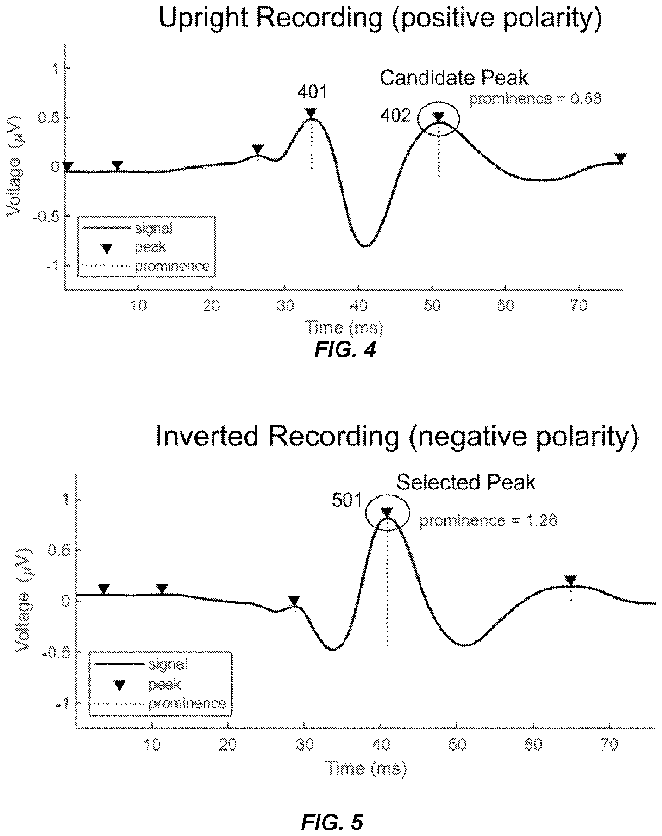

The method of Figure 3 is described below with reference to Figures 4 and 5, which are graphs of a monitorable baseline SSEP potentials in an upright representation (Figure 4) and an inverted representation (Figure 5). It is to be understood that the implementation shown in Figure 3 and the baseline recording depicted in Figures 4 and 5 are very specific for exemplary purposes only, such that other implementations and other baseline recordings are possible and are within the scope of the disclosure.

At step 3-1, the SSEP system 100 identifies candidate peaks in the upright representation and the inverted representation. Note that the baseline recording can have several candidate peaks. For example, six candidate peaks are identified in Figure 4, and five candidate peaks are identified in Figure 5. A goal is to identify which one of these candidate peaks is most prominent.

At step 3-2, for each candidate peak identified at step 3-1, the SSEP system 100 calculates the prominence of that peak. To calculate the prominence, the SSEP system 100 first extends a horizontal line from the candidate peak to the left and right until the line either (a) crosses the signal because there is a higher peak or (b) reaches the left or right end of the signal. In some implementations, the horizontal line extended from the peak for the prominence calculation is further limited by an analysis range. Next, the SSEP system 100 finds a minimum of the signal in each of the two intervals defined above. This point is either a local minima or one of the signal endpoints. Finally, the SSEP system 100 determines prominence based on the vertical distance between the peak and the higher of the two interval minima.

At step 3-3, the candidate peaks are be filtered based on an analysis range. The analysis range is a range of expected latencies where the evoked potential is likely to occur. The analysis range may be based on the stimulation site as there is a known approximate relationship between distance an evoked potential must travel through the nervous system between stimulation and recording site (the longer the distance traveled, the longer the expected latency). There is a range of expected latencies due to natural variability in subjects anatomy, neurophysiology and pathophysiology. The analysis range for a given stimulation site may be set from values in the literature, expert opinion or learned from previous subject data. In some implementations, the analysis range may be a function of the evoked potential responses from other stimulation or recording channels. Note that the filtering of candidate peaks by analysis range may occur before the calculation of the prominence in step 3-2.

In this way, the SSEP system 100 identifies all positive and negative extrema (also known as "peaks and troughs" or "local maxima and local minima") and calculates the prominence for each. The extrema with the greatest prominence within the analysis range is selected for both the "upright recording" and the "inverted recording" and their prominence values are compared to each other as shown in Figures 4 and 5.

At step 3-4 the SSEP system 100 selects the candidate peak with greatest prominence as the primary peak. In the illustrated example, the candidate peak 501 with greatest prominence is identified in Figure 5 as having a prominence of 1.26. Note that this peak is actually a trough, or local minima, in the baseline SSEP potential because the recording is in the inverted representation in Figure 5. The prominence of other candidate peaks as represented by the vertical lines can be seen to be smaller.

In this way, a marking algorithm identifies all positive and negative extrema (also known as "peaks and troughs" or "local maxima and local minima") within the analysis range and calculates the prominence for each. For positive extrema, the peak finding and prominence calculations use the waveform as recorded. When calculating the prominence for the troughs, the waveform is inverted, and the prominence calculation proceeds as described above. The extrema with the greatest prominence within the analysis range is selected for both the "upright waveform" and the "inverted waveform" and their prominence values are compared to each other as shown in Figures 4 and 5. Of these two candidate peaks, the local maxima or local minima with the greatest prominence is selected for marker placement.

By calculating the prominence of both positive and negative extrema, the primary peak could be identified as either a peak or a trough. When the peak marker is placed at a local minima, or trough, (i.e. placed at a local *maxima* for the *inverted* version of the recording), the peak is said to have a negative polarity. When the peak marker is placed at a local maxima, the peak has a positive polarity.

At step 3-5, onset and offset peaks 401-402 (see Figure 4) are identified on either side of the most prominent peak 501. These onset and offset peaks 401-402 are of the opposite polarity of the most prominent peak 501. The onset peak 401 is identified in a window of time leading up to the most prominent peak 501, and the offset marker 402 is identified in a window of time following the most prominent peak 501. The extrema in the onset and offset window with the largest absolute value difference in voltage from the peak marker is selected as the onset peak and offset peak, respectively. In other implementations, only extrema immediately adjacent to the primary peak are considered potential reference peaks.

At step 3-6, the onset or offset peak 401-402 that maximizes the amplitude to the most prominent peak 501 is selected as the reference peak. In other implementations, slope, second derivative or other evoked potential morphology features may be used to select the reference peak. Furthermore, in some implementations, markers used to determine amplitude and latency could be placed on both the onset and offset peaks 401-402.

In some implementations, new peak markers are placed on each updated SSEP ensemble average to track the amplitude and latency of the evoked potential throughout a surgery. Peak marking on the ongoing waveform is similar to the method described above. However, adjustments can be made to provide consistent markings between the baseline and ongoing waveforms. Specifically, in the ongoing waveform case, the polarity of the baseline epoch marking and the decision to mark to the onset or offset of the baseline epoch are enforced on the ongoing waveform. Furthermore, the analysis range can be recentered around the most prominent peak in the baseline waveform. In some implementations, the analysis range for the ongoing waveform peak marking could be asymmetric since it is more likely for the latency to be prolonged than to shorten.

### Determining Monitorability of the Baseline SSEP Waveform

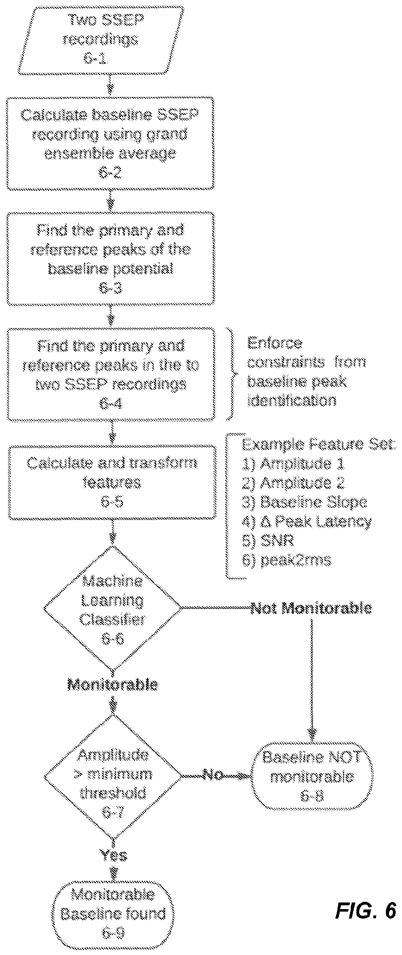

Referring now to Figure 6, shown is a flowchart of a method of determining whether a baseline recording contains a monitorable baseline potential. This method is an example implementation for steps 2-1 to 2-3 of the method described above in relation to Figure 2. Thus, much like the method of Figure 2, the method of Figure 6 can be executed by an SSEP system, for example the SSEP system 100 depicted in Figure 1. In particular implementations, the method is executed by the NIDD 104 of the SSEP system 100. More generally, the method of Figure 6 can be executed by any suitable SSEP system.

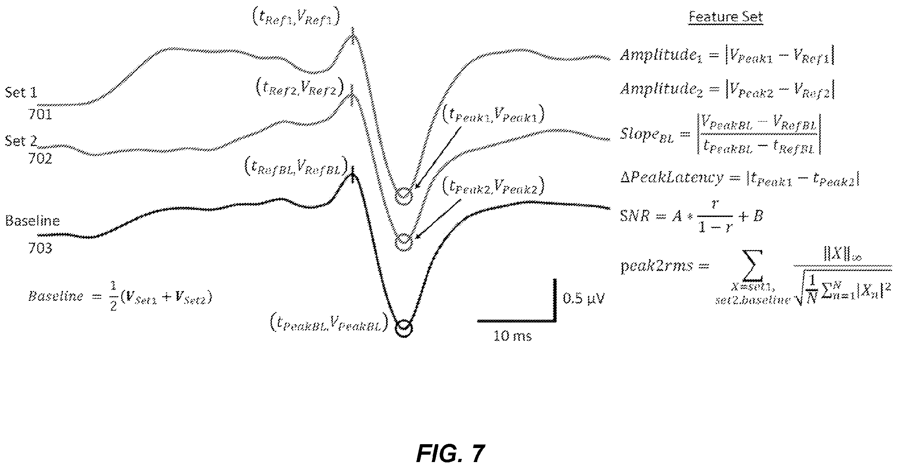

The method of Figure 6 is described below with reference to Figure 7, which is a graph of baseline recordings. It is to be understood that the implementation shown in Figure 6 and the baseline recordings depicted in Figure 7 are very specific for exemplary purposes only, such that other implementations and other baseline recordings are possible and are within the scope of the disclosure.

At step 6-1, the SSEP system 100 acquires two independent sets of SSEP recordings. Each set includes a number of non-overlapping SSEP single trials, for example 280 non-overlapping SSEP single trials or other suitable number of non-overlapping SSEP single trials. In some implementations, each set includes the same number of non-overlapping SSEP single trials. Other implementations are possible. For each set, the SSEP system 100 averages the SSEP single trials to produce a recording representing the set. Thus, two SSEP recordings are produced.

At step 6-2, the SSEP system 100 produces a baseline recording representing the two sets, for example by averaging all of the SSEP single trials or by averaging the two baseline SSEP recordings. For example, if each independent set is an average of 280 single trials, a grand-ensemble average can be an average of all 560 single trials. As a result, as shown in Figure 7, the SSEP system 100 produces three recordings, including a first SSEP recording 701 for the first set, a second SSEP recording 702 for the second set, and a third recording 703 which is the baseline SSEP recording.

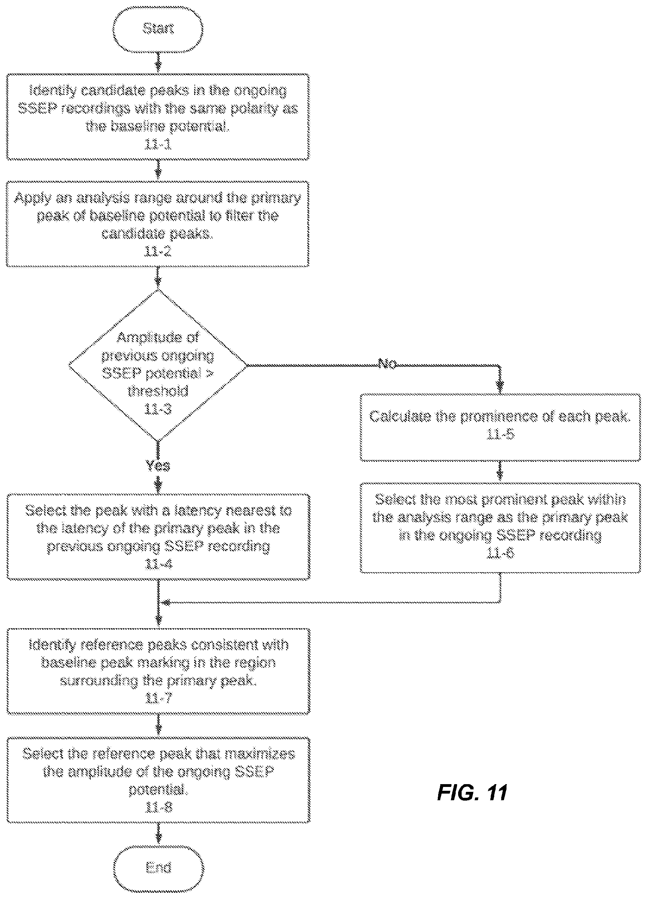

At steps 6-3 and 6-4, the SSEP system 100 finds the primary and reference peaks for potentials in the three recordings 701-703 produced at step 6-2. Peak finding in the baseline SSEP recording 703 at step 6-3 is performed as described above and illustrated in Figures 3-5. The amplitude between the peak and reference markers in the baseline SSEP potential is retained to compare against the minimum amplitude threshold at a later step. Peak finding in the two sets 701-702 is performed with the ongoing peak finding method described below in Figure 11. This is done to enforce consistent marker placement on the two sets by using the same polarity and marking to the onset/offset as in the baseline SSEP potential.

At step 6-5, the SSEP system 100 calculates features based on the SSEP recordings and the identified peaks. There are many possibilities for the features. For example, as shown in Figures 6 and 7, the features can include an amplitude of the potential in the first recording 701, an amplitude of the potential in the second recording 702, the slope between the primary peak and reference markers of the potential in the baseline recording 703, an absolute value of the difference in primary peak latency of the potentials in the first and second recordings 701-702, a SNR (signal to noise ratio) value calculated from first and second recordings 701-702, and a measure of the peak amplitudes to the RMS (root mean square) of each recording 701-703. Additional and alternative features are also possible.

In some implementations, features may derived from the peak marking algorithms applied at steps 6-3 and 6-4 or from additional processing performed on the recordings 701-703. The first two features in the example feature set described above and in Figures 6 and 7 are the individual potential amplitude values for each of the two sets 701-702. The amplitude is defined as the absolute value difference in voltage between the primary and reference peaks. The third feature is the absolute value of the slope between the primary and reference peaks of the baseline SSEP potential. The fourth feature is the absolute value difference between the peak latencies or the timing of the peak markers from the potentials in each set 701-702. The fifth feature is a measure of the SNR, for example SNR as defined by Coppola [Coppola R, Tabor R, Buchsbaum MS. Signal to noise ratio and response variability measurements in single trial evoked potentials. Electroencephalograph and Clinical Neurophysiology, 1978, 44: 214-222.].

This feature is computed by first finding the correlation coefficient (r) between the two sets that comprise the baseline recording. This correlation is taken to be the 'signal' and it is divided by (1-r) which is the noise estimate. This feature can also be thought of as a measure of inter-set variability. The sixth feature is another type of SNR measure derived from the ratio of the peak amplitude to the RMS of the recording. This is calculated for all three recordings 701-703 and summed together. Finally, transformations can be applied to the feature values to adjust for skewed feature distributions. This can improve classification performance and/or interpretability for certain types of machine learning classification algorithms. Other feature sets are possible. Features that quantify the morphology, repeatability, or SNR of the recordings 701-703 are good candidates for features.

In some implementations, the SSEP system 100 implements a machine learning classifier at step 6-6 to determine if there is a monitorable baseline SSEP potential based on the features calculated at step 6-5. The machine learning classifier is a supervised machine learning model used for binary classification. In an exemplary implementation, an SVM (Support Vector Machine) is used as the machine learning classifier. Alternative supervised machine learning algorithms such as gradient boosted decision trees, random forest, logistic regression, or a neural network could be used in other implementations. In some implementations, for example if a deep learning neural network framework is applied, specific features do not need to be engineered.

In some implementations, the machine learning classifier has been trained to classify the baseline potential as either monitorable or non-monitorable based on a database of labeled baseline examples and their corresponding features. Recordings in this database could be adjudicated as either monitorable or non-monitorable by expert clinical evaluators. In other implementations, the machine learning algorithm may apply a multiclass classification approach rather than a binary classification approach. One possible example of the classes used for multiclass classification could be monitorable, identifiable but not monitorable, and not identifiable.

In some implementations an additional criteria is applied after the machine learning classification. For example, if at step 6-7 the minimum amplitude threshold is not exceeded, then at step 6-8 the baseline potential is deemed to be not monitorable. In such case, more single trails can be collected and the SSEP system 100 can try again to find a monitorable baseline potential. However, if at step 6-7 the minimum amplitude threshold is exceeded, then at step 6-9 the baseline potential is deemed to be monitorable. There are many possibilities for the minimum amplitude threshold. The minimum amplitude threshold can for example be 0.2 µV or some other suitable value.

In some implementations, the algorithm is agnostic to the stimulation and recording site. All features in the example feature set described in Figures 6 and 7 are agnostic to the stimulation/recording site since the stimulation/recording site specific parameters (e.g. analysis range) can be applied at the stage of the peak marking algorithm. This allows the machine learning classifier to be trained on labeled data originating from any stimulation/recording site. In other implementations, the features or training data may be specific to a particular stimulation and recording site pair. Additionally, the minimum amplitude threshold may be different for different stimulation and/or recording sites.

In some implementations, setting the baseline is a static process in which two sets of data (e.g. 280 single trials each, 560 total single trials) are collected and processed by the classification algorithm. If the classification algorithm determines there is not monitorable potential the system may enter a non-monitorable state for that SSEP channel. In some implementations, the SSEP system 100 will collect a third set of SSEP single trials and try to set a baseline using sets 2 and 3.

In some implementations, if the confidence (positive class probability) that the baseline is monitorable exceeds a high threshold (e.g. 98%), then the SSEP system 100 can set the baseline early.

In some implementations, a user has an ability to override the algorithmically determined baseline classification. If the initial baseline waveform is negatively classified, and the user has not yet reset or overridden the baseline, then a second attempt is made to positively classify the baseline. In this instance, a third ensemble of 280 single trials is acquired, the second and third ensembles yield a new super-ensemble which is then re-classified. If a second negative-classification occurs, then the baseline is considered 'unmonitorable'.

In some implementations, the supervised machine learning algorithm is trained using data from an annotated clinical database. Waveforms in this database can be adjudicated as either monitorable or non-monitorable by expert clinical evaluators. Each waveform in the database can be paired with the subsequent waveform to simulate the two independent sets of data used for baseline classification. Features can then be calculated on those paired waveforms as described above, and the class label of monitorable applied to a feature sample if both of the underlying waveforms were adjudicated as monitorable.

In some implementations, data in the annotated database is taken from Saphenous and Tibial stimulation sites. However, the algorithm is montage agnostic as the feature set does not depend on the montage. All features in the feature set are derived from the peak markings, and montage specific parameters can be applied at the stage of the peak marking algorithm. In some implementations, the machine learning algorithm is trained on all the available data, and resulting support vectors are saved off and stored in the SSEP system 100 or other location to classify data in use.

In other configurations, other methods can be used to identify peaks. For example, the peak marker is placed on the most prominent extrema (peak or trough) within the analysis range. The onset marker is placed on the maximum extrema of opposite polarity of the peak marker within a window leading up to the peak marker. To calculate the peak marking derived features, the peak and onset marker are first placed on the super-ensemble average that would constitute the baseline waveform. The amplitude of the markers on the super-ensemble baseline waveform is retained to compare against the minimum amplitude threshold. The polarity of the baseline peak (i.e. whether a peak (positive polarity) or trough (negative polarity) is the most prominent peak) is retained to help determine five of the features for the classification. Peak and onset markers are then placed on the two independent sets and the polarity is forced to match that of the baseline waveform. The analysis window for placing peaks marker on the two independent sets is extended if the baseline peak marker is near the edge of the analysis window. The length of this extension is a function of the width of the baseline analysis window and how close the peak is to the edge of that window.

### Automated Optimization of the Baseline SSEP Recording

In preferred implementations, the SSEP system 100 has a baseline optimization state where collecting the baseline recordings is adaptive. An advantage of machine learning classification algorithms is that they can provide not just a classification, but also a probability for that classification that represents how confident the algorithm is in the classification. This and other computed values (e.g. amplitude or SNR) provide the ability to optimize or improve the baseline collection phase of SSEP monitoring in ways not obvious or available to the typical neuromonitoring technologist.

A common measure of a binary classification algorithms' confidence in a result it has provided is the positive class probability. The positive class probability represents the likelihood assigned by the trained model that a sample belongs in the positive class. Positive class probabilities typically range from 0 to 1, where 0 indicates the model is very confident a sample belongs in the negative class, and 1 indicates the model is very confident the sample belongs in the positive class. Some machine learning models output these probabilities natively (e.g. neural networks). For other types of models the positive class probability must be calculated post-hoc (e.g. by applying Platt scaling to SVM scores). For the classification task described above and outlined in Figure 6 - determining if a baseline SSEP waveform is monitorable or non-monitorable - the positive class probability (or monitorable class probability) is the probability assigned to a sample by the trained model that the baseline SSEP potential in monitorable.

An advantage of the approach disclosed herein is the ability for the SSEP system 100 to periodically (e.g. every 10 collected trials) split all collected single trials into two sets and perform a comparison using the machine learning classifier. By periodically evaluating the monitorability of the collected data, the SSEP system 100 can establish the baseline early if the data is determined to of excellent quality or take steps to improve the signal quality if the recorded data is non-monitorable. This approach provides clinical advantages to the SSEP system 100.

In a preferred implementation, the SSEP system 100 evaluates the baseline SSEP potential after 280, 420, and 560 collected single trials. Other frequencies of evaluating the baseline are possible. If the result from 280 or 420 single trials returns a positive class probability from the machine learning classifier that exceeds a high threshold (e.g. 0.98 or 98%), the SSEP system 100 can establish an early baseline. If the system does not set an early baseline, the nominal threshold for positive class probability of 0.5 (or 50%) is applied after collecting 560 single trials.

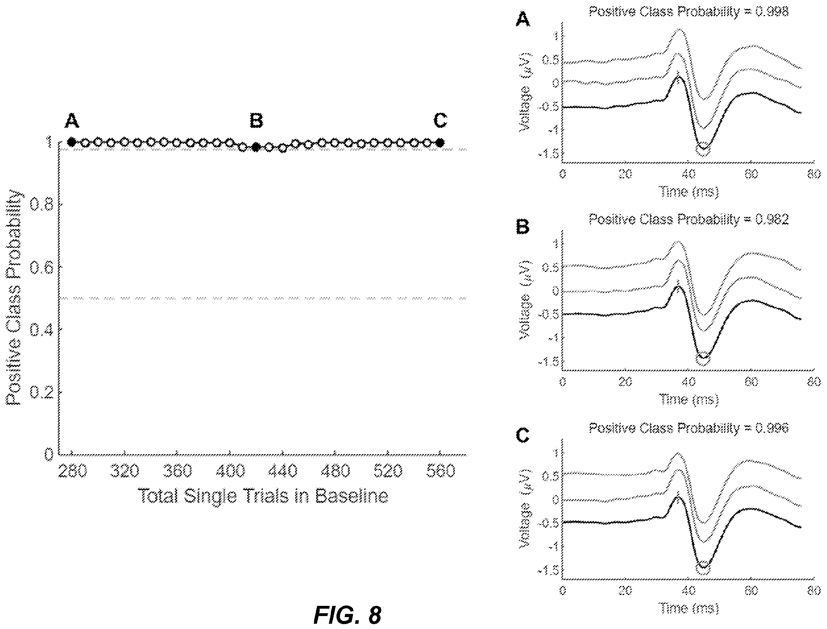

Referring now to Figure 8, shown is a collection of charts relating to a monitorable example with excellent quality data depicting the scenario where the SSEP system 100 could set an early baseline. In the main chart, the data points show the positive class probability if the SSEP system 100 had evaluated the monitorability of the baseline every 10 samples. The filled data points indicate the results of the classification algorithm at the preferred evaluating frequency (after 280, 420, and 560 collected single trials). The upper dashed line delineates a high threshold for the positive class probability at 0.98 (or 98%). The lower dashed line is the nominal threshold of 0.5 (or 50%). The charts 8A, 8B, and 8C display the two independent sets and their resulting baseline (with peak and reference markers) when evaluating the data after 240, 420, and 560 single trials, respectively.

This case example demonstrates very high positive class probability (>0.98) even with few single trials collected. With very high positive class probability, it may be possible to conclude that there is a monitorable baseline potential with a relatively small number of single trials SSEP data and the baseline could be established faster. The baseline provided for monitoring if set after 280 single trials (8A) is virtually indistinguishable from and would provide equivalent monitoring as the baseline set after 560 single trials (8B). In a preferred embodiment, the baseline would be set after collecting 280 single trials for this example.

In some implementations, if the result from 280 or 420 single trials returns a positive class probability from the machine learning classifier that is low, indicating the possibility of obtaining a non-monitorable classification after 560 single trials, steps to improve the signal could be taken automatically. For example, the SSEP system 100 could increase the stimulus intensity, increase the stimulus duration, decrease the stimulation frequency, increase the number of single trials to be ensemble averaged, or change signal processing parameters such as filter settings or mother wavelet for denoising.

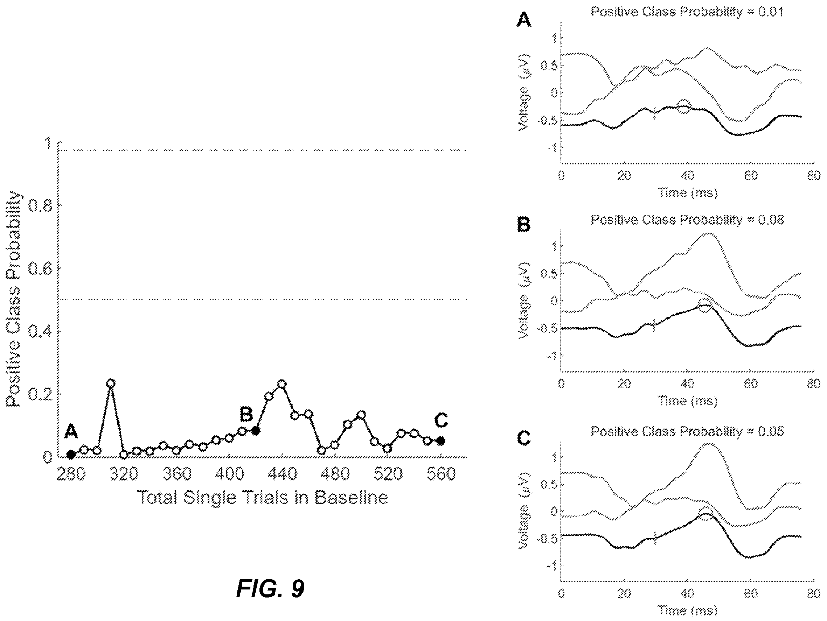

Referring now to Figure 9, shown is a collection of charts relating to a non-monitorable example depicting the scenario where the SSEP system 100 could trigger enhancements to the SSEP collection parameters and signal processing. In this case example, the positive class probability is very low (0.01 or 1%) after 280 single trials and improvement is not seen over time. If a static baseline approach was being followed, monitoring of this channel may not provide meaningful clinical information. In some implementations, identifying a low positive class probability early in the baseline collection period (e.g. after 280 or fewer single trials) triggers changes to the collection parameters such as increasing the stimulus intensity, increasing the stimulus duration, or changing the signal processing (e.g. filtering). In some implementations, signal processing optimizations may be applied retroactively (i.e. to data already collected) as the system is able to store the pre-processed SSEP recordings and the single trials. In this manner the SSEP system 100 is more likely to obtain a monitorable baseline than if a static baseline collection approach had been followed.

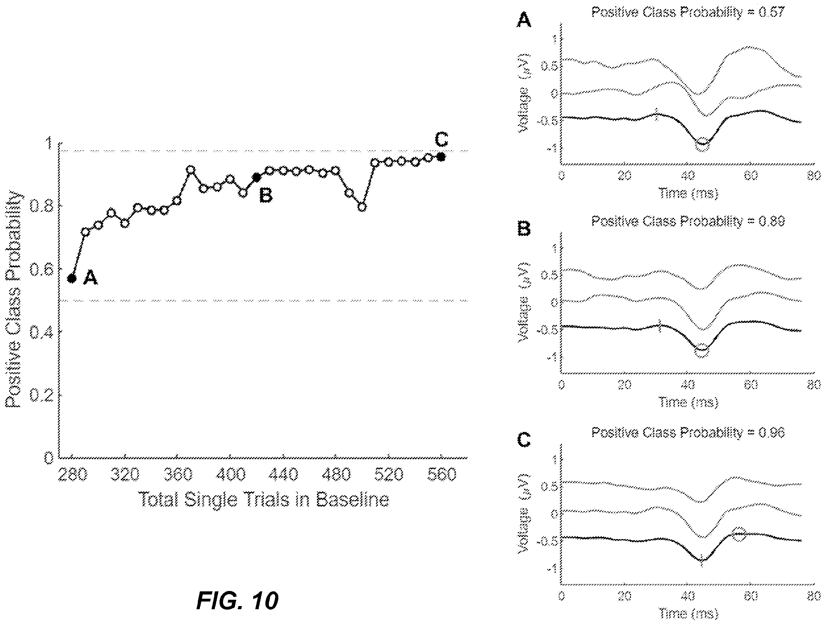

Referring now to Figure 10, shown is a collection of charts relating to a monitorable example with improving positive class probability as more single trials are collected. The case example demonstrates initial positive class probability of roughly 57% but improves to roughly 96%. Compared to the example in Figure 8, concluding that there is a monitorable baseline potential may involve a larger number of single trials of SSEP data. While the baseline in Figure 10 was monitorable, in some implementations it may still benefit from improvements during the SSEP baseline collection period including increasing stimulus intensity and/or the others listed above.

In some implementations, a user has an ability to override the algorithmically determined baseline classification. If the initial baseline potential is negatively classified, the user may override that non-monitorable classification and enable monitoring for that channel. The positive class probability can aid users deciding whether to override a decision of the baseline classification algorithm. If a user overrides a properly classified non-monitorable baseline, this can result in very poor monitoring and unreliable information for the user.

In some implementations, attempting to override a non-monitorable baseline prompts the SSEP system 100 to display the confidence information to the user when they try to override the baseline (e.g. "Are you sure you want to monitor this baseline? The algorithm is 95% confident this baseline is not monitorable"). There are many alternative messages and ways to display this information to the user through. This message may only be raised if the positive class probability indicates the system is very confident in the classification.

The positive class probability computed by the machine learning classifier when the baseline is set is also used in some implementations to select which stimulation and recording channels to display and provide alerting information to the user. For example, depending on the recording montage, there may be multiple channels of cranial information (e.g. CPz-FPz and CP3-CP4). Frequently, one channel may have better quality signal than the other which would provide improved monitoring (improved sensitivity and specificity). The positive class probability can be used to select one of those for ongoing monitoring or weigh the contributions of each lead to the alert status.

The improvement and optimization processes described above rely on the positive class probability provided by the machine learning classifier. In other implementations, the improvement and optimization processes could be driven by other values derived from the SSEP data (e.g. amplitude or SNR) independent of or in conjunction with the positive class probability.

### Identifying Peaks in the Ongoing SSEP Recordings

Referring now to Figure 11, shown is a flowchart of a method of identifying the relevant peaks of an evoked potential in an ongoing SSEP recording. This method is an example implementation for step 2-5 of the method described above in relation to Figure 2. Thus, much like the method of Figure 2, the method of Figure 11 can be executed by an SSEP system, for example the SSEP system 100 depicted in Figure 1. In particular implementations, the method is executed by the NIDD 104 of the SSEP system 100. More generally, the method of Figure 11 can be executed by any suitable SSEP system.

Identifying the primary and reference peak within the ongoing SSEP recording is an important step in monitoring with a NIDD 104 as it allows the amplitude and latency of the evoked potential response to determined and compared to those found in the baseline waveform and raise an alert if the alert criteria have been met. To ensure the comparison to the baseline potential is accurate, information from the baseline peak marking must be applied when identifying peaks in the ongoing SSEP recordings. Additionally, to ensure the same region of the evoked potential is being compared, this method does not rely solely on the prominence of the peak, but tracks the primary peak of the ongoing potential over time.

At step 11-1, the SSEP system 100 identifies the candidate peaks in the ongoing recording with the same polarity as the primary peak of the potential in the baseline SSEP recording. If the primary peak of the baseline SSEP potential is found to be a local maxima then the primary peaks in the subsequent ongoing SSEP recordings will be a local maxima. Similarly, if the primary peak of the baseline SSEP potential is found to be a local minima then the primary peaks in the subsequent ongoing SSEP recordings will be a local minima.

At step 11-2, the candidate peaks are filtered based on an analysis range. The analysis range for ongoing peak marking is a function of the latency of the primary peak of the baseline SSEP potential. This analysis range or window can be a percentage of the latency of the baseline or a fixed amount of time. In the preferred implementation, the ongoing analysis range is asymmetrical since it is more likely for the latency to be prolonged than to shorten during monitoring and it extends from 17% prior to the primary peak to 30% beyond the primary peak. In some implementations the ongoing peak finding analysis range may depend on the stimulation or recording site. Other ongoing peak finding analysis ranges are possible within the scope of this disclosure.

At step 11-3, the amplitude of the potential in the previous ongoing SSEP recording is compared to a threshold. In some implementations this threshold is 25% of the baseline SSEP potential amplitude. Other thresholds are possible, and the threshold may be a function of the noise floor or SNR.

When the amplitude exceeds the threshold, it is expected that the evoked potential is still present in the response, even if diminished. Under these conditions a peak tracking approach is preferred. At step 11-4, this tracking approach is applied by selecting as the primary peak, the candidate peak that is closest in latency to the primary peak of the potential from the previous ongoing SSEP recording.

If the amplitude is below the threshold in 11-3, it is possible the evoked potential is no longer present or noise may be as large or larger than the response. In this case the approach is similar to the baseline peak marking algorithm. The prominence of each peak is calculated in step 11-5, and the peak with the greatest prominence within the ongoing analysis range is selected as the primary peak in step 11-6.

At step 11-7, potential reference peaks are identified in the region around the primary peak. The reference peak of the ongoing potential must be the same direction relative to the primary peak as the reference peak of the baseline SSEP potential. For example, if the reference peak in the baseline is an onset (prior to the primary peak), this must hold true in the ongoing potential.

Finally, at step 11-8, the reference that maximizes the amplitude of the ongoing SSEP potential is selected as the reference. Other methods for selecting the reference peak are possible. For example, the reference peak may be the peak of the opposite polarity closest to the primary peak in the direction specified by the baseline peak marking.

### Case Examples

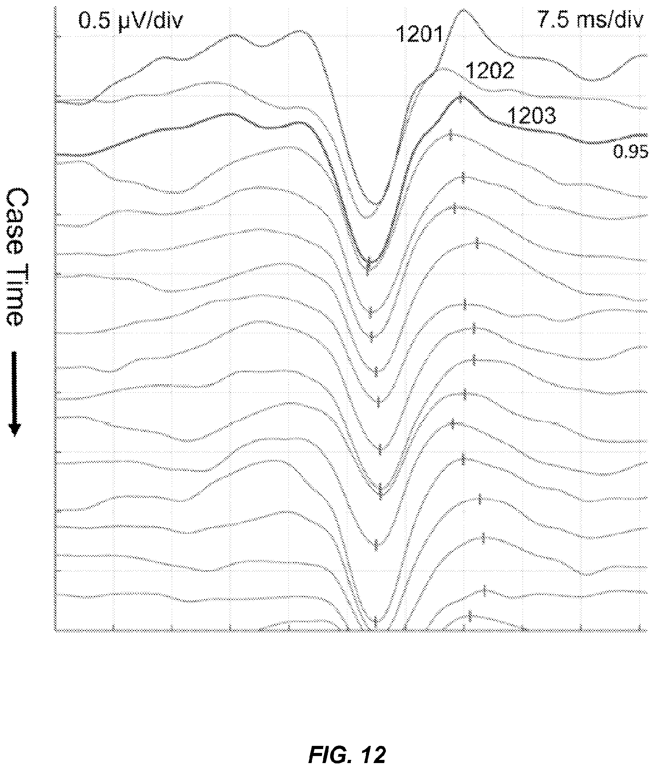

Referring now to Figure 12, shown is a graph of baseline and ongoing SSEP recordings for a case example with a monitorable baseline potential. This way of looking at the data is called a "waterfall" view. It shows independent sets (non-overlapping ensemble averages) stacked atop each other. In this case, each recording is the ensemble average of 280 single trials collected at 3.1Hz stimulation (~90 seconds of data collection). Case time progresses from the beginning of the case at the top of the figure and the last recording collected in the case would be at the bottom of the figure. The data here is viewed through a tool to view case data retrospectively.

The top two recordings 1201 and 1202 are the two sets used to form the baseline. The third recording is the baseline recording 1203. In the first example, the evoked potential is very clear. Also included is the positive class probability near the right of the baseline. Here it is 0.95 (95%), indicating that the algorithm is very confident the baseline is monitorable. Below the baseline are the ongoing SSEP recordings. The amplitude and latency of the potential in the ongoing SSEP recordings are stable and similar to baseline and there is no alert for a significant change.

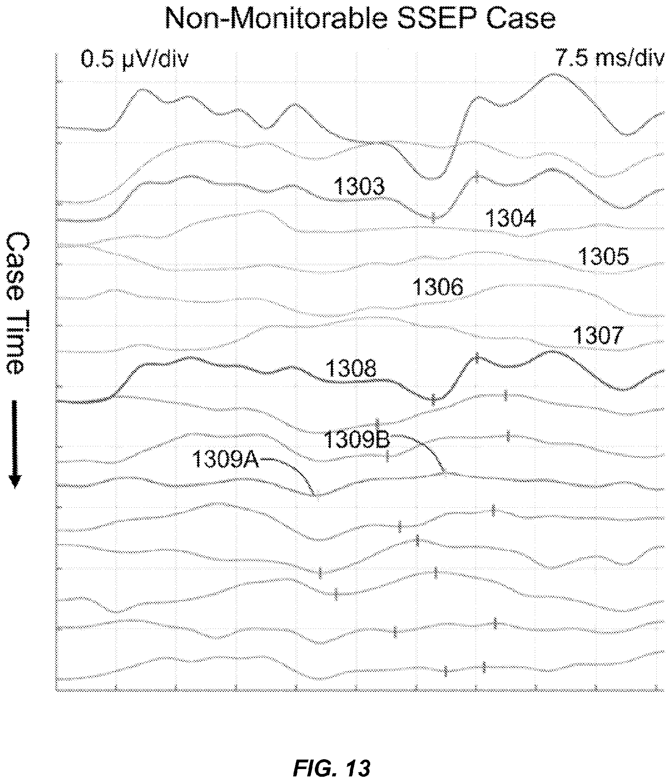

Referring now to Figure 13, shown is a graph of baseline and ongoing SSEP recordings for a case example with a non-monitorable baseline potential. In this example, the baseline recording 1303 (thicker third recording down with the markers) is classified as non-monitorable. While it may appear that there is an evoked potential response in the baseline, the baseline classification algorithm is confident the baseline recording 1303 does not contain a monitorable potential with a 2% positive class probability. The next four ongoing SSEP recordings 1304-1307 are grey because this channel is in a state indicating no monitorable baseline. Then the user overrides the baseline classification (the thicker line 1308 about halfway down). The channel moves into and alert state (see markers 1309A and 1309B) quickly and the markers in the ongoing trace are not actually marking an evoked potential. The monitoring after the override is not providing reliable information to the user. Notifying the user of the low likelihood of a baseline waveform given by the algorithm may prevent them from improperly overriding the classification as demonstrated here.

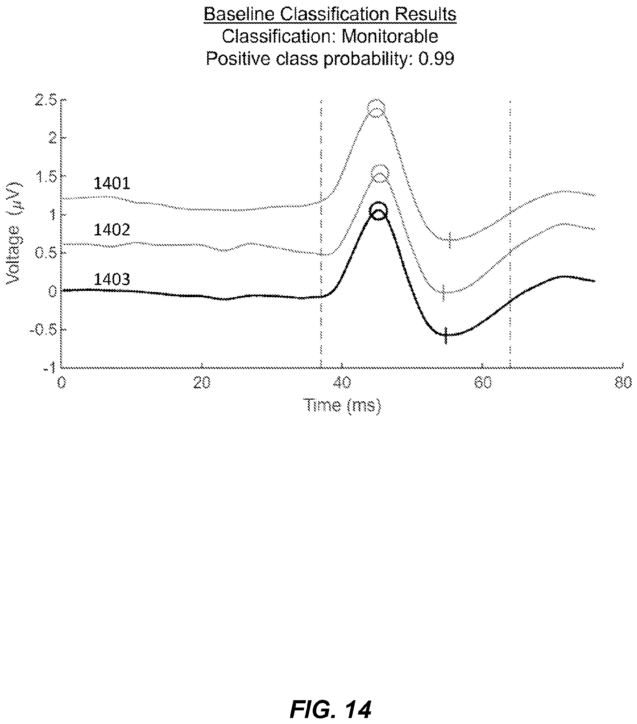

Referring now to Figure 14, shown is a graph of SSEP recordings relating to a case example with a monitorable baseline potential. The SSEP recordings include a first SSEP recording 1401 for a first set, a second SSEP recording 1402 for a second set, and a third recording 1403 which is the baseline SSEP recording. The vertical dashed lines delineate the analysis range for the baseline peak marking. The potentials in the SSEP recordings 1401-1403 are consistent with one another with expected morphology, and the case example demonstrates a 99% positive class probability.

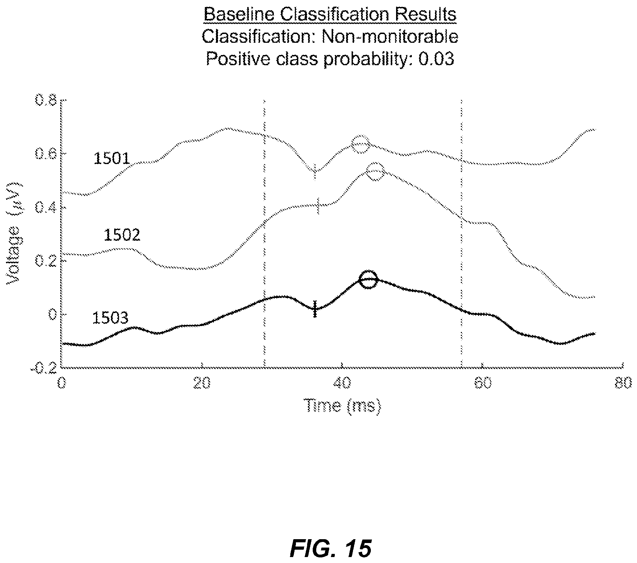

Referring now to Figure 15, shown is a graph of SSEP recordings relating to a case example with a non-monitorable baseline potential. The SSEP recordings include a first SSEP recording 1501 for a first set, a second SSEP recording 1502 for a second set, and a third recording 1503 which is the baseline SSEP recording. The SSEP recording s 1501-1503 are not very consistent with one another, and the case example demonstrates only a 3% positive class probability.

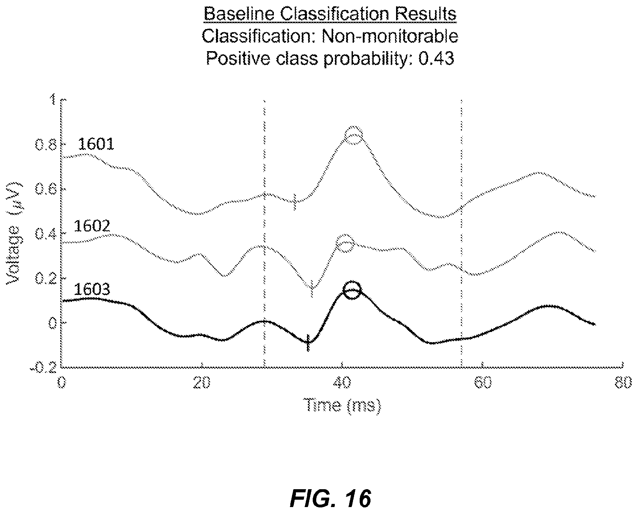

Referring now to Figure 16, shown is a graph of SSEP recordings relating to a case example where it is not entirely clear if the baseline potential is monitorable or non-monitorable. The SSEP recordings include a first SSEP recording 1601 for a first set, a second SSEP recording 1602 for a second set, and a third recording 1603 which is the baseline SSEP recording. The SSEP recordings 1601-1603 are noisy and only somewhat consistent with one another, and the case example demonstrates a 43% positive class probability, which could potentially be a false negative classification.

Numerous modifications and variations of the present disclosure are possible in light of the above teachings.

Embodiment 1: A method for execution by an SSEP (Somatosensory Evoked Potentials) system, comprising: acquiring at least one SSEP recording from a patient; determining or calculating presence and characteristics of a monitorable baseline SSEP potential based on the at least one SSEP recording; acquiring ongoing SSEP recordings from the patient; comparing the ongoing SSEP potential to the monitorable baseline potential; and upon the ongoing SSEP potential deviating from the monitorable baseline potential according to a defined criteria, executing an alert.

Embodiment 2: The method of Embodiment 1, wherein: acquiring at least one SSEP recording comprises acquiring two SSEP recordings based on two corresponding independent sets of SSEP data; and the monitorable baseline potential is calculated based on a grand ensemble average of the independent sets of SSEP data when the baseline SSEP potential is deemed monitorable based on features of the two SSEP potentials identified from the two independent sets of SSEP data as well as the potential identified from the grand ensemble average.

Embodiment 3: The method of Embodiment 2, further comprising: calculating the features of the two SSEP potentials, wherein the features comprise: an amplitude of a first potential of the two SSEP recordings, an amplitude of a second potential of the two SSEP recordings, an absolute value of a difference in amplitude between the first and second potentials, an absolute value of a difference in onset latency between the first and second potentials, an absolute value of a difference in peak latency between the first and second potentials, and an SNR (signal to noise ratio) of the first and second potentials in comparison to the entire respective recordings.

Embodiment 4: The method of any one of Embodiments 1-4, wherein calculating the features comprises calculating peak/trough markers of each baseline SSEP potential by: identifying candidate peaks in an upright representation and an inverted representation of the baseline SSEP recordings; and identifying which candidate peak has greatest prominence based on how much the candidate peak stands out due to its intrinsic height and its location relative to other candidate peaks.

Embodiment 5: The method of any one of Embodiments 0 to 0, comprising: determining whether the baseline epoch potential is deemed monitorable using an SVM (support vector machine) which classifies the baseline epoch potential as either monitorable or not monitorable based on the features of the two SSEP potentials.

Embodiment 6: The method of any of Embodiments 1-6, further comprising: determining whether the baseline epoch potential is deemed monitorable using a wavelet convolution neural network which classifies the baseline epoch potential as either monitorable or not monitorable based on the features of the two SSEP potentials.

Embodiment 7: The method of Embodiment 0 or Embodiment 0, further comprising: determining a confidence value of whether the baseline epoch potential is monitorable.

Embodiment 8: The method of Embodiment 0, comprising: adapting size of the two corresponding independent sets of SSEP data depending on the confidence value.

Embodiment 9: The method of any one of Embodiments 0 to 0, comprising: for each ongoing SSEP recording, calculating peak/trough markers of the ongoing SSEP potential by: identifying candidate peaks in an upright representation and an inverted representation of the ongoing SSEP recording; and identifying which candidate peak has greatest prominence based on how much the candidate peak stands out due to its intrinsic height and its location relative to other candidate peaks; wherein the candidate peak with greatest prominence is used in the comparing of the ongoing SSEP potential to the monitorable baseline potential.

Embodiment 10: The method of Embodiment 0, wherein: the defined criteria comprises a defined decrease in amplitude based on decreased prominence and/or a defined increase in latency based on delay of peak.

Embodiment 11: The method of one of Embodiments 0 to 0, wherein the alert comprises an auditory alert, a visual alert, and/or a haptic alert.

Embodiment 12: The method of one of Embodiments 0 to 0, further comprising: identifying artifacts on the ongoing SSEP recordings and potentials due to presence of anesthesia in the patient and/or noise from a surrounding environment; and compensating for the artifacts to mitigate unnecessary alerting.

Embodiment 13: A non-transitory computer readable medium having recorded thereon statements and instructions that, when executed by a processor of an SSEP (Somatosensory Evoked Potentials) system, configure the processor to implement a method according to any one of Embodiments 0 to 0.

Embodiment 14: An SSEP (Somatosensory Evoked Potentials) system, comprising: recording electrodes configured to generate electric recordings of a patient's nervous system; recording electrodes configured to sense the electric potentials generated by stimulation upon their traversing the nervous system; a nerve injury detection device coupled to the recording electrodes and the recording electrodes and configured to implement a method according to any one of Embodiments 0 to 0.

Embodiment 15: The SSEP system of Embodiment 0, wherein the nerve injury detection device comprises: a processor; and a non-transitory computer readable medium having recorded thereon statements and instructions that, when executed by the processor of the SSEP system, configure the processor to implement the nerve injury detection device.

Embodiment 16: A method comprising a step or any combination of steps as described and/or depicted herein.

Embodiment 17: A non-transitory computer readable medium having recorded thereon statements and instructions that, when executed by a processor of an apparatus, configure the processor to implement a method comprising a step or any combination of steps as described and/or depicted herein.

Embodiment 18: An apparatus comprising a component or any combination of components as described and/or depicted herein.

Embodiment A: A method for determining or calculating the presence, absence and monitorability of an SSEP (Somatosensory Evoked Potentials) potential within one or more SSEP recordings, comprising: acquiring at least one SSEP recording from a subject; analyzing the at least one SSEP recording by evaluating the prominence of peaks contained in it; determining or calculating presence and characteristics of a monitorable baseline SSEP potential based on the at least one SSEP recording; acquiring ongoing SSEP recordings from the subject; comparing the ongoing SSEP potential to the monitorable baseline potential; and upon the ongoing SSEP potential deviating from the monitorable baseline potential according to a defined criteria, executing an alert.

Embodiment B: The method of Embodiment A:, wherein: acquiring at least one SSEP recording comprises acquiring two SSEP recordings based on two independent sets of SSEP data; and the monitorable baseline SSEP potential is calculated based on a grand ensemble average of the independent sets of SSEP data when the baseline SSEP potential is deemed monitorable based on features of the SSEP recordings, the two SSEP potentials identified from the two independent sets of SSEP data as well as the potential identified from the grand ensemble average.

Embodiment C: The method of Embodiment B:, further comprising: calculating the features of the two SSEP potentials, wherein the features comprise: an amplitude of a first potential of the two SSEP recordings, an amplitude of a second potential of the two SSEP recordings, an absolute value of the slope between the primary and reference peaks in the baseline potential, an absolute value of a difference in peak latency of the potentials in the first and second SSEP recordings, a SNR (signal to noise ratio) of the first and second potentials in comparison to the entire respective recordings, and a ratio of the peak amplitude of the potentials to the RMS (root mean square) of the entire recordings for the two SSEP recordings and the grand ensemble baseline recording.

Embodiment D: The method of Embodiment C, wherein calculating the features comprises calculating peak/trough markers of each baseline SSEP potential by: identifying candidate peaks in an upright representation and an inverted representation of the baseline SSEP recordings; and identifying which candidate peak has greatest prominence based on how much the candidate peak stands out due to its intrinsic height and its location relative to other candidate peaks.

Embodiment E: The method of any one of Embodiments B through D, comprising: determining whether the baseline SSEP potential is deemed monitorable using a machine learning classification algorithm which classifies the baseline SSEP potential as either monitorable or non-monitorable based on the features of the two SSEP potentials.

Embodiment F: The method of Embodiment B, further comprising: determining whether the baseline SSEP potential is deemed monitorable using a wavelet convolution neural network which classifies the baseline potential as either monitorable or not monitorable based without engineering features of the two SSEP potentials.

Embodiment G: The method of Embodiment E or F, further comprising: determining a confidence value of whether the baseline potential is monitorable.

Embodiment H: The method of Embodiment G, comprising: adapting size of the two corresponding independent sets of SSEP data depending on the confidence value.

Embodiment I: The method of any one of Embodiments A through H, comprising: for each ongoing SSEP recording, calculating peak/trough markers of the ongoing SSEP potential by: identifying candidate peaks in an upright representation or an inverted representation of the ongoing SSEP recording depending on the polarity of the baseline SSEP potential; and comparing the amplitude of the potential in the previous ongoing SSEP recording to a threshold; selecting the candidate peak based on which peak has the latency nearest the latency of the potential in the previous ongoing SSEP potential or which has greatest prominence based on how much the candidate peak stands out due to its intrinsic height and its location relative to other candidate peaks depending on the amplitude comparison to the previous ongoing SSEP potential; wherein the selected peak with is used in the comparing of the ongoing SSEP potential to the monitorable baseline potential.

Embodiment J: The method of Embodiment I, wherein: the defined criteria comprises a defined decrease in amplitude based on decreased prominence and/or a defined increase in latency based on delay of peak.

Embodiment K: The method of one of Embodiments A through J, wherein the alert comprises an auditory alert, a visual alert, and/or a haptic alert.

Embodiment L: The method of one of Embodiments A through K, further comprising: identifying artifacts on the ongoing SSEP recordings and potentials due to presence of anesthesia in the subject and/or noise from a surrounding environment; compensating for the artifacts to mitigate unnecessary alerting.

Embodiment M: A non-transitory computer readable medium having recorded thereon statements and instructions that, when executed by a processor of an SSEP (Somatosensory Evoked Potentials) system, configure the processor to implement a method according to any one of Embodiments A through L.

Embodiment N: An SSEP (Somatosensory Evoked Potentials) system, comprising: stimulating electrodes configured to generate electric responses from a subject's nervous system; recording electrodes configured to sense the electric potentials generated by stimulation upon their traversing the nervous system; a nerve injury detection device coupled to the recording electrodes and the recording electrodes and configured to implement a method according to any one of Embodiments A through L.

Embodiment O: The SSEP system of Embodiment N, wherein the nerve injury detection device comprises: a processor; and a non-transitory computer readable medium having recorded thereon statements and instructions that, when executed by the processor of the SSEP system, configure the processor to implement the nerve injury detection device.

Embodiment P: A method comprising a step or any combination of steps as described and/or depicted herein.

Embodiment Q: A non-transitory computer readable medium having recorded thereon statements and instructions that, when executed by a processor of an apparatus, configure the processor to implement a method comprising a step or any combination of steps as described and/or depicted herein.

Embodiment R: An apparatus comprising a component or any combination of components as described and/or depicted herein.

Numerous modifications and variations of the present disclosure are possible in light of the above teachings. It is to be understood that the implementation described herein is for SSEPs but application to other methods of generating evoked potentials such as visual evoked potentials or brainstem auditory evoked potentials is possible. Within the scope of the appended claims, the disclosure may be practised otherwise than as specifically described herein.

### Further aspects

In a first broad independent aspect, an embodiment provides a method for determining the presence, absence, and/or monitorability of an evoked potential within one or more SSEP recordings, comprising:
acquiring at least one SSEP recording from a subject;
determining presence and characteristics of the evoked potential in the at least one SSEP recording to determine presence of a monitorable baseline potential;
acquiring ongoing SSEP recordings from the subject to determine ongoing evoked potentials;
comparing the ongoing evoked potentials to the monitorable baseline potential; and
upon the ongoing evoked potentials deviating from the monitorable baseline potential according to a defined criteria, executing an alert.

In a subsidiary aspect, the method further comprises:
acquiring at least one SSEP recording comprises acquiring two independent SSEP recordings comprising a first and a second SSEP recording, and identifying two SSEP evoked potentials, including a first evoked potential from the first SSEP recording and a second evoked potential from the second SSEP recording;
determining a baseline recording based on a grand ensemble average of the first and second of the two independent SSEP recordings; and
determining whether the baseline recording is monitorable based on features of the two independent SSEP recordings, two evoked potentials identified from the two independent sets of SSEP recordings, and/or the potential identified from the grand ensemble average.

In a further subsidiary aspect, the method further comprises
calculating the features of the first evoked potential and the second evoked potential, wherein the features comprise:
an amplitude of a first potential of the two SSEP recordings,
an amplitude of a second potential of the two SSEP recordings,
an absolute value of a slope between the primary and reference peaks in the baseline potential,

an absolute value of a difference in peak latency of the potentials in the first and second SSEP recordings,
a SNR (signal to noise ratio) of the first and second potentials in comparison to an entire respective recordings, and
a ratio of a peak amplitude of the potentials to the RMS (root mean square) of the entire respective recordings for the two SSEP recordings and the grand ensemble baseline recording.

In a further subsidiary aspect, the method comprises calculating the features comprises calculating peak/trough markers of each baseline SSEP potential by:
identifying candidate peaks in an upright representation and an inverted representation of the baseline SSEP recordings; and
identifying which candidate peak has greatest prominence based on how much the candidate peak stands out due to its intrinsic height and its location relative to other candidate peaks, and assigning the candidate peak with the greatest prominence as the primary peak.

In a further subsidiary aspect, the method further comprises the step of identifying onset and offset peaks surrounding the reference peak, and
selecting an onset or offset peak that maximizes the amplitude of the potential as the reference peak of the baseline potential.

In a further subsidiary aspect, the method comprises
determining whether the baseline recording is deemed monitorable using a machine learning classification algorithm which classifies the baseline SSEP potential as either monitorable or non-monitorable based on the features of the two SSEP evoked potentials.

In a further subsidiary aspect, the method further comprises determining whether the baseline SSEP potential is deemed monitorable using a wavelet convolution neural network which classifies the baseline potential as either monitorable or not monitorable based without engineering features of the two SSEP potentials.

In a further subsidiary aspect, the method further comprises determining a confidence value of whether the baseline potential is monitorable.

In a further subsidiary aspect, the method comprises adapting size of the two corresponding independent sets of SSEP data depending on the confidence value.

In a further subsidiary aspect, the method comprises
for each ongoing SSEP recording, calculating peak/trough markers of the ongoing SSEP recordings by:
identifying candidate peaks in an upright representation or an inverted representation of the ongoing SSEP recording depending on a polarity of the baseline SSEP potential; and
   comparing an amplitude of the potential in the previous ongoing SSEP recording to a threshold;
selecting the candidate peak based on which peak has the latency nearest the latency of the potential in the previous ongoing SSEP potential or which has greatest prominence based on how much the candidate peak stands out due to its intrinsic height and its location relative to other candidate peaks depending on the amplitude comparison to the previous ongoing SSEP potential;
wherein the selected peak with is used in the comparing of the ongoing SSEP potential to the monitorable baseline potential.

In a further subsidiary aspect, the defined criteria comprises a defined decrease in amplitude based on decreased prominence and/or a defined increase in latency based on delay of peak.

In a further subsidiary aspect, the alert comprises an auditory alert, a visual alert, and/or a haptic alert.

In a subsidiary aspect, the method further comprises
identifying artifacts on the ongoing SSEP recordings and potentials due to presence of anesthesia in the subject and/or noise from a surrounding environment;
compensating for the artifacts to mitigate unnecessary alerting.

In a further aspect, an embodiment provides a non-transitory computer readable medium having recorded thereon statements and instructions that, when executed by a processor of an SSEP (Somatosensory Evoked Potentials) system, configure the processor to implement a method according to the first broad aspect.

In a further aspect, an embodiment provides an SSEP (Somatosensory Evoked Potentials) system, comprising:
stimulating electrodes configured to generate electric responses from a subject's nervous system;
recording electrodes configured to sense the electric potentials generated by stimulation upon their traversing the nervous system;
a nerve injury detection device coupled to the recording electrodes and the recording electrodes and configured to implement a method according to claim 1.

In a further subsidiary aspect, the nerve injury detection device comprises:
a processor; and
a non-transitory computer readable medium having recorded thereon statements and instructions that, when executed by the processor of the SSEP system, configure the processor to implement the nerve injury detection device.

In further subsidiary aspect, the method comprises determining whether the baseline recording is deemed monitorable by identifying primary and reference peaks of the two SSEP potentials.

In a further aspect, an embodiment provides a method of identifying one or more relevant peaks of an evoked potential in an ongoing SSEP recording, the method comprising the steps of:
identifying one or more candidate peaks in an ongoing SSEP recording with the same polarity as a primary peak of an evoked potential in a predetermined baseline SSEP recording;
filtering the one or more candidate peaks abased on an analysis range;
comparing an amplitude of the evoked potential in a previous ongoing SSEP recording to a threshold;
when the amplitude exceeds the threshold, applying a peak tracking by selecting as the primary peak the candidate peak that is closest in latency to the primary peak of the potential from the previous ongoing SSEP recording;
selecting the peak with the greatest prominence within an ongoing analysis range as the primary peak;
identifying potential reference peaks in a region around the primary peak; and
selecting a reference that maximizes the amplitude of the ongoing SSEP evoked potential.

## Claims

1. An SSEP (Somatosensory Evoked Potentials) system, comprising:
stimulating electrodes (103) configured to generate electric responses from a subject's nervous system;
recording electrodes (102) configured to sense the electric potentials generated by stimulation upon their traversing the nervous system;
a nerve injury detection device (104) coupled to the recording electrodes (102) and the stimulating electrodes (103), wherein the nerve injury detection device (104) is configured to:
acquiring at least two independent SSEP recordings from a subject; the two independent SSEP recordings comprising a first SSEP recording and a second SSEP recording;
determining whether a baseline recording, derived from a grand ensemble average of the first and second SSEP recordings, is monitorable based on features of the two independent SSEP recordings and the baseline recording; and, if the baseline recording is determined to be monitorable:
acquiring ongoing SSEP recordings from the subject to determine ongoing evoked potentials;
comparing the ongoing evoked potentials to the monitorable baseline potential; and
upon the ongoing evoked potentials deviating from the monitorable baseline potential according to a defined criteria, executing an alert.

2. The system of claim 1, wherein:
acquiring at least one SSEP recording comprises acquiring two independent SSEP recordings comprising a first and a second SSEP recording, and identifying two SSEP evoked potentials, including a first evoked potential from the first SSEP recording and a second evoked potential from the second SSEP recording;
determining a baseline recording based on a grand ensemble average of the first and second of the two independent SSEP recordings; and
determining whether the baseline recording is monitorable based on features of the two independent SSEP recordings, two evoked potentials identified from the two independent sets of SSEP recordings, and/or the potential identified from the grand ensemble average.

3. The system of claim 1 or claim 2, wherein the method further comprises:
calculating the features of the first evoked potential and the second evoked potential, wherein the features comprise:
an amplitude of a first potential of the two SSEP recordings,
an amplitude of a second potential of the two SSEP recordings,
an absolute value of a slope between the primary and reference peaks in the baseline potential,
an absolute value of a difference in peak latency of the potentials in the first and second SSEP recordings,
a SNR (signal to noise ratio) of the first and second potentials in comparison to an entire respective recordings, and
a ratio of a peak amplitude of the potentials to the RMS (root mean square) of the entire respective recordings for the two SSEP recordings and the grand ensemble baseline recording.

4. The system of claim 3, wherein calculating the features comprises calculating peak/trough markers of each baseline SSEP potential by:
identifying candidate peaks in an upright representation and an inverted representation of the baseline SSEP recordings; and
identifying which candidate peak has greatest prominence based on how much the candidate peak stands out due to its intrinsic height and its location relative to other candidate peaks, and assigning the candidate peak with the greatest prominence as the primary peak.

5. The system of claim 3 or claim 4, wherein the method further comprises identifying onset and offset peaks surrounding the reference peak, and
selecting an onset or offset peak that maximizes the amplitude of the potential as the reference peak of the baseline potential.

6. The system of any one of claims 2 through 5, wherein the method further comprises:
determining whether the baseline recording is deemed monitorable using a machine learning classification algorithm which classifies the baseline SSEP potential as either monitorable or non-monitorable based on the features of the two SSEP evoked potentials.

7. The system of any one of claims 2 through 6, wherein the method further comprises:
determining whether the baseline SSEP potential is deemed monitorable using a wavelet convolution neural network which classifies the baseline potential as either monitorable or not monitorable based without engineering features of the two SSEP potentials.

8. The system of claim 6 or claim 7, wherein the method further comprises:
determining a confidence value of whether the baseline potential is monitorable.

9. The system of claim 8, wherein the method further comprises:
adapting size of the two corresponding independent sets of SSEP recordings depending on the confidence value.

10. The system of any one of claims 1 through 9, wherein the method further comprises:
for each ongoing SSEP recording, calculating peak/trough markers of the ongoing SSEP recordings by:
identifying candidate peaks in an upright representation or an inverted representation of the ongoing SSEP recording depending on a polarity of the baseline SSEP potential; and
comparing an amplitude of the potential in the previous ongoing SSEP recording to a threshold;
selecting the candidate peak based on which peak has the latency nearest the latency of the potential in the previous ongoing SSEP potential or which has greatest prominence based on how much the candidate peak stands out due to its intrinsic height and its location relative to other candidate peaks depending on the amplitude comparison to the previous ongoing SSEP potential;
wherein the selected peak with is used in the comparing of the ongoing SSEP potential to the monitorable baseline potential.

11. The system of claim 10, wherein:
the defined criteria comprises a defined decrease in amplitude based on decreased prominence and/or a defined increase in latency based on delay of peak.

12. The system of any one of claims 1 through 11, wherein the alert comprises an auditory alert, a visual alert, and/or a haptic alert.

13. The system of any one of claims 1 through 12, wherein the method further comprises:
identifying artifacts on the ongoing SSEP recordings and potentials due to presence of anesthesia in the subject and/or noise from a surrounding environment;
compensating for the artifacts to mitigate unnecessary alerting.

14. A non-transitory computer readable medium having recorded thereon statements and instructions that, when executed by a processor of the system of claim 1, configure the processor to implement the method comprising: acquiring at least two independent SSEP recordings from a subject, the two independent SSEP recordings comprising a first SSEP recording and a second SSEP recording; determining whether a baseline recording, derived from a grand ensemble average of the first and second SSEP recordings, is monitorable based on features of the two independent SSEP recordings and the baseline recording; and, if the baseline recording is determined to be monitorable: acquiring ongoing SSEP recordings from the subject to determine ongoing evoked potentials; comparing the ongoing evoked potentials to the monitorable baseline potential; and upon the ongoing evoked potentials deviating from the monitorable baseline potential according to a defined criteria, executing an alert.
